# EUROPEAN PATENT SPECIFICATION

(11) **EP 1 917 995 B1**
(45) Date of publication and mention of the grant of the patent: **23.06.2010**
(21) Application number: 07254276.4
(22) Date of filing: 29.10.2007
(51) Int. Cl.: A61M 25/00, A61M 25/01, B24C 1/10

(54) **Stylet having a roughened outer surface**
Mandrin mit aufgerauter äußerer Oberfläche
Stylet ayant une surface extérieure rugosifiée

(30) Priority: 30.10.2006 US 863508 P
(43) Date of publication of application: 07.05.2008
(73) Proprietor: Codman & Shurtleff, Raynham, MA 02767 (US)
(72) Inventor: Schorn, Greg M., Milford, MA 01757 (US)
(74) Representative: Tunstall, Christopher Stephen

(56) References cited:
- DE-A1- 1 566 583
- US-A- 4 636 200

## Description

### FIELD OF THE INVENTION

### Background of the Invention

The use of a stiffening member or stylet within a flexible catheter to stiffen the catheter to help guide the introduction of the catheter into the desired location in a patient is well known. Conventionally once the catheter is in place, the stylet is removed and discarded.

Catheters are often made of low durometer silicone. For example, ventricular catheters are often made of low durometer (about 50A) silicone, and have a predetermined outer diameter, inner diameter and length. Such a ventricular catheter is often provided preloaded with a polished stainless steel round stylet. However, neurosurgeons have frequently complained that they experience great difficulty when removing the stylet from the catheter due to friction between the stylet and catheter, which can cause, among other things, displacement of the distal end of the catheter from the target site, and/or cutting of the catheter.

To reduce the friction, attempts have been made to coat the stylet with PTFE or modify the geometrical shape so that it has a square cross-section. Others still have tried to modify the manufacturing process to decrease the amount of so called stickiness of the catheter by either including a post curing process or compounding the catheter with a filler, such as barium sulfate.

However, surgeons have been generally dissatisfied with these attempts as they are accustomed to a conventional catheter feel and design. For example, surgeons have complained that a square-shaped stylet does not provide them with the same placement feel as a conventional round stylet.

Accordingly, there remains a need for a stylet that can be preloaded in a conventional catheter, such as, for example, a low durometer catheter, and can provide sufficient stiffness to the catheter to assist a user in guiding the catheter to its desired location in a patient while thereafter permitting the stylet to be easily removed from the catheter.

DE 1 566 583 discusses a stylet and a method of treating a stylet.

It is also an aspect of the invention to provide a stylet design that is simple to produce and that can be made of standard materials by convenient and cost effective procedures.

In light of the present disclosure and the practice of the present invention, other advantages and solutions to other problems will become apparent to those skilled in the relevant art.

### SUMMARY OF THE INVENTION

The present invention generally provides a method of treating a stylet according to claim 1 that has a proximal end, a distal end and an outer surface. The stylet is substantially cylindrical. The outer surface of the stylet is treated, or roughened, by a glass peening operation, such that its maximum profile peak height is greater than 30µm, its roughness average is greater than 5µm, and its root mean square roughness is greater than 8µm. This results in reducing the removal force of the treated stylet from a silicone catheter.

### Brief Description of the Drawings

The invention will be more fully understood from the following detailed description taken in conjunction with the accompanying drawings, in which:

FIG. 1 is a side view of a test method for removing a stylet from a catheter, according to the present invention;

FIG. 2 is a cross-sectional view of the stylet only taken along lines 2-2 of Figure 1 and looking in the direction of the arrows;

FIG. 3 is a chart illustrating the stylet removal force of a conventional stylet and a roughened stylet according to the present invention; and

FIG. 4 is a chart showing various roughness properties of a conventional stylet and a roughened stylet according with the present invention.

### DETAILED DESCRIPTION OF THE INVENTION

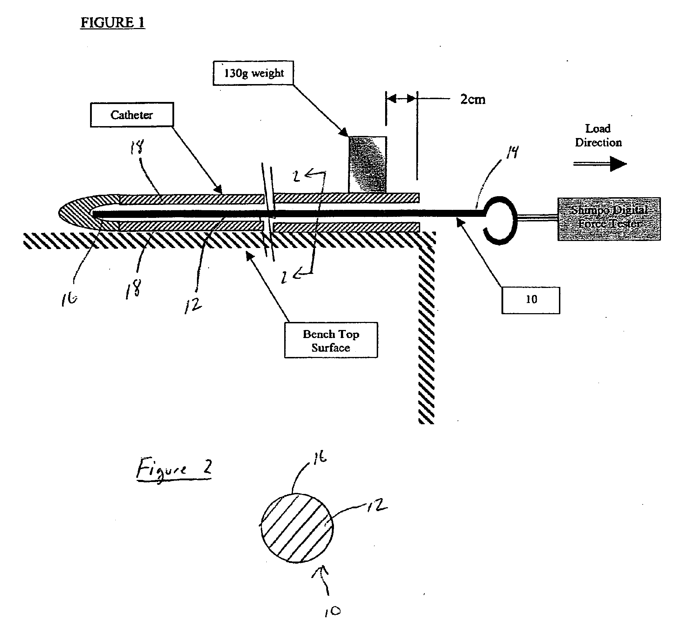

Referring now to Figures 1 and 2, the present invention generally provides a stylet 10 that has an elongate stylet body 12 having a proximal end 14, a distal end and an outer surface 16. For the sake of clarity in the drawings, only the cross-section of the stylet is shown in Figure 2. The stylet is substantially cylindrical at least for a majority of its length, and preferably for its entire length. Outer surface 16 is treated, or roughened, preferably by a glass peening or a bead blasting operation, such that its maximum profile peak height is greater than 30µm, its roughness average is greater than 5µm, and its root mean square roughness is greater than 8µm.

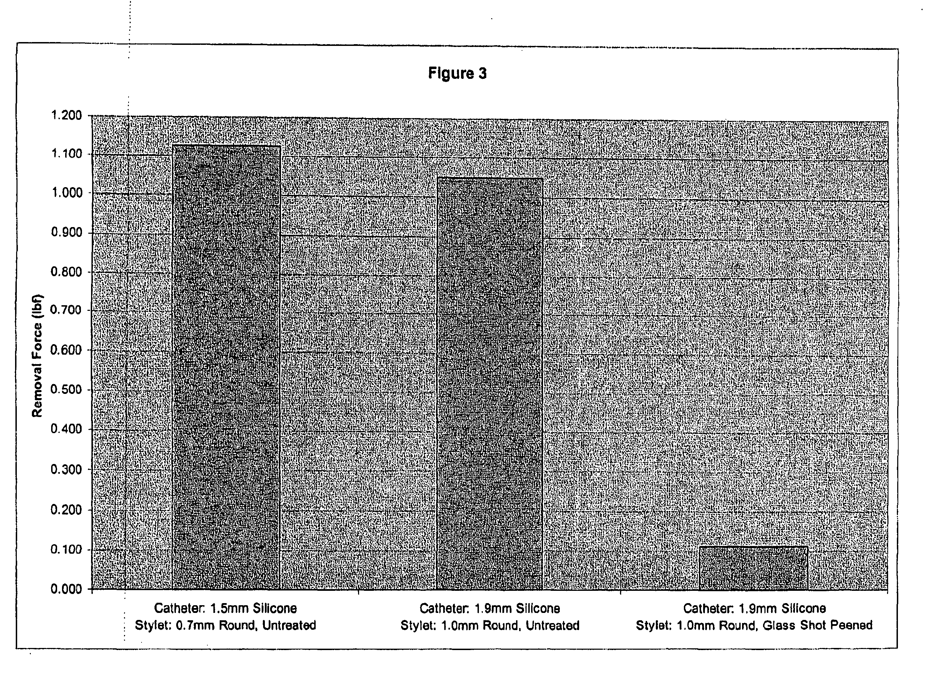

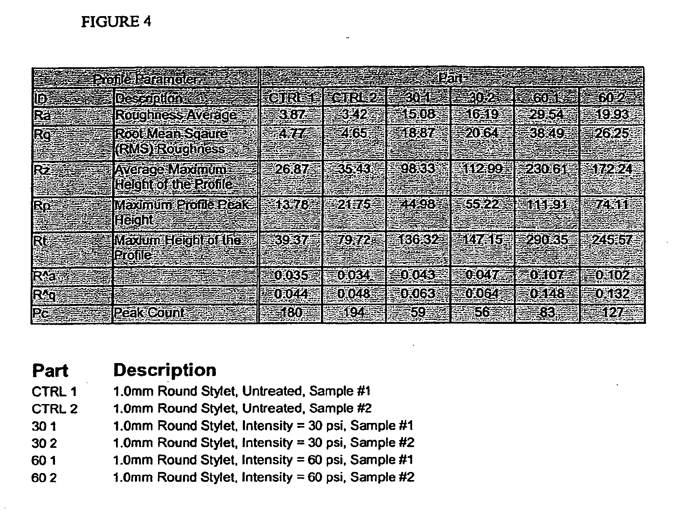

Surface treatment is typically provided by conventional shot peening. As one skilled in the art is readily aware, in shot peening, metal or glass shot is bombarded against the surface of the component with suitable intensity and overlapping coverage. The conventional use for shot peening is to reduce the potential for stress cracking on metal parts by creating a compressed layer at the surface. The present inventors use the shot peening process to roughen the outer surface of the stylet. U.S. Pat. No. 5,057,108 issued to Shetty et al. discloses a process of stainless steel shot blasting, glass bead blasting, electropolishing, and passivation. In addition, a method of surface finishing a medical device shield using metallic media is described in U.S. Patent No. 5,673,473 to Johnson, et al. According to a method of the invention stylet 10 is subject to a glass shot peening for at least 10 minutes with a glass shot size of about 100µm and with an intensity range between 206,84 - 413,68 KPa (30-60 psi). This results in the stylet 10 being essentially 100% treated along the majority of the stylet's length. The test results shown in Figures 3 and 4 included stylets treated in this manner.

Stylet 10 is preferably preloaded in a low durometer catheter 18. When preloaded, the stylet provides sufficient stiffness to the catheter to assist a user in guiding the catheter to its desired location in a patient. Thereafter, because the outer surface of stylet 10 has been roughened, stylet 10 is easily removed from the catheter 18 by a user. In a currently preferred exemplary embodiment, stylet 10 is made of stainless steel, and catheter 18 is made of silicone.

Referring now to Figure 1, a test method is illustrated, which was used to compare the removal force of a catheter according to the present invention as compared to a conventional stainless steel polished round stylet. In accordance with this test method, the following steps are followed:
1. Place catheter with loaded stylet onto a flat bench top surface 20 such that the proximal end of the catheter extends over the edge of the table.
2. Place a 130g weight approximately 2cm from the proximal end of the catheter. (This is to consistently hold the catheter in place during the test without compressing the ID against the stylet.)
3. Connect a digital force gauge to the stylet (e.g., a Shimpo Digital Force Tester, 0-10 N)
4. Tare the force gauge and set for peak hold; and
5. Extract the stylet from the catheter and record the peak load required to remove it from the catheter.

Following this test procedure, the stylet removal force according to the present invention for a 1.0 mm round stylet is about 0,44 N (0,1 lbf), whereas for a conventional round stylet, which is 0.7 mm round or 1.0mm round, the removal force is about 4,89 N (1,1 lbf) as shown on Figure 3. Thus, the removal force is about a magnitude of order less when using a stylet that has been treated in accordance with the present invention.

Referring now to Figure 4, a chart showing various roughness properties of a conventional stylet (CTRL 1 and CTRL 2) and a roughened stylet in accordance with the present invention (30 1, 30 2, 60 1, and 60 2). The properties were measured in accordance with ANSI B46.1: 1985 and ISO 4287: 1996.

One skilled in the art will appreciate further features and advantages of the invention based on the above-described embodiments. Accordingly, the invention is not to be limited by what has been particularly shown and described, except as indicated by the appended claims.

## Claims

1. A method of treating a stylet (10) so that it can be easily removed from a catheter (18), wherein the catheter is made of an elastomeric material, and the stylet is made of a rigid material, the stylet having an elongate stylet body (12) having a proximal end (14), a distal end and an outer surface (16), said stylet being substantially cylindrical at least for a majority of its length, the method being **characterised in that** it comprises the steps of:
subjecting the stylet body to a glass shot peening process,
wherein the subjecting step continues for at least 10 minutes with a glass shot size of about 100 µm and with an intensity range between 207-414 kPa (30-60 psi).

2. The method of treating a stylet according to claim 1, wherein the subjecting step achieves essentially 100% coverage along the majority of the stylet's length.

## Patentansprüche

1. Verfahren zum Behandeln eines Mandrins (10), so dass er leicht von einem Katheter (18) entfernt werden kann, wobei der Katheter aus einem elastomeren Material hergestellt ist und der Mandrin aus einem starren Material hergestellt ist, wobei der Mandrin einen länglichen Mandrinkörper (12) mit einem proximalen Ende (14), einem distalen Ende und einer Außenfläche (16) hat, wobei der Mandrin wenigstens über einen Hauptteil seiner Länge im Wesentlichen zylindrisch ist, wobei das Verfahren **dadurch gekennzeichnet ist, dass** es die Schritte aufweist:
Einbringen des Mandrinkörpers in einen Kugelstrahlprozess,
wobei der Einbringschritt wenigstens 10 Minuten dauert, mit einer Glaskugelgröße von ungefähr 100 µm und mit einem Intensitätsbereich zwischen 207-414 kPa (30-60 psi).

2. Verfahren zum Behandeln eines Mandrins nach Anspruch 1, bei dem der Schritt des Einbringens eine im Wesentlichen 100%ige Abdeckung über den Hauptteil der Länge des Mandrins erreicht.

## Revendications

1. Procédé pour traiter un stylet (10) de sorte qu'il peut être facilement retiré d'un cathéter (18), dans lequel le cathéter est réalisé avec un matériau élastomère, et le stylet est réalisé avec un matériau rigide, le stylet ayant un corps de stylet allongé (12) ayant une extrémité proximale (14), une extrémité distale et une surface externe (16), ledit stylet étant sensiblement cylindrique au moins sur une majeure partie de sa longueur, le procédé étant **caractérisé en ce qu'**il comprend les étapes consistant à :
soumettre le corps de stylet à un procédé de martelage par billes de verre,
dans lequel l'étape de soumission continue pendant au moins 10 minutes avec une taille de bille de verre d'environ 100 µm et avec une intensité comprise entre 207 et 414 kPa (30-60 psi).

2. Procédé pour traiter un stylet selon la revendication 1, dans lequel l'étape de soumission atteint essentiellement 100 % de la couverture le long de la majeure partie de la longueur du stylet.
